# EUROPEAN PATENT APPLICATION

(11) **EP 3 241 488 A1**
(43) Date of publication of application: **08.11.2017**
(21) Application number: 16382202.6
(22) Date of filing: 06.05.2016
(51) Int. Cl.: A61B 3/113, A61B 5/11

(54) **DEVICE FOR SYNCHRONIZED MEASUREMENT OF OCULAR AND CEPHALIC MOVEMENT**

(71) Applicant: Aura Innovative Robotics, S.L, 28033 Madrid (ES)
(72) Inventor: García Cena, Cecilia, 28012 Madrid (ES); Gómez Andrés, David, 28036 Madrid (ES); Pulido Valdeoliva, Irene, 28036 Madrid (ES); Acebrón López, Rafael, 28034 Madrid (ES); Espinoza Gómez, Ricardo, 28055 Madrid (ES); Ramos Vázquez, Sara, 28010 Madrid (ES)
(74) Representative: Manuel Illescas Asociados, S.L.

(57) **Abstract**

Device for synchronized measurement of eye and head movements of a subject (1) comprising: a helmet (2); a U-shaped arm (3a, 3b) attached to the helmet through passive joints (7a, 7b), in whose central portion (3b) a camera (5) and a lighting system (6) are arranged; a motion sensor (4) provided with fixation means (14) and disposed in the glabellar region of the subject (1); a emitter (10) of visual stimuli sequences; a signal hub (8); and processing means (9) that receives images taken by the camera (5) and the data collected by the motion sensor (4) and quantifies the cephalic movement and eye movements made by the subject (1).

## Description

### FIELD OF THE INVENTION

This invention belongs to the technical field of bioengineering and biomedical devices. More specifically, the invention relates to a device for synchronized measurement of eye(s) and cephalic movements of a subject in order to determine possible abnormalities of such movements. To that end, in the device according to the invention, the subject must follow some specific visual stimuli or his/her head is subjected to a series of movements while the resulting head and eyes movements are recorded independently.

The abnormal eye-cephalic movements are clear indicators of alterations in different areas of nervous systems like vestibular system, brain stem, cerebellum, basal ganglia, or in some cortical areas as "frontal eye field" (brain region involved in controlling eye movements). The object of this invention is to provide functional information of the operation of the various parts of the nervous system.

### BACKGROUND OF THE INVENTION

The proper functioning of ocular movements of human being depends on his/her ability to perceive and interpret the received information, the ability to have a clear objective and a planning to achieve it as well as the proper functioning of the system that transmits orders and controls the correct execution of said movements. Several neural systems, which interact among them, control and suited the functioning of the ocular and ocular-cephalic movements. Cortical networks, basal ganglia, some specialized brain stem nuclei and the vestibular system are organized to produce different eye movements that maintain the position of maximum visual acuity on the images or objects of interest. This is essential for the daily life of all human beings: we need all these regions of the central nervous system, which are highly evolved, to function properly. Whereas this highly evolved system of control and is highly complex, it is logical to expect that appear abnormalities in ocular movements as a result of the existence of injury or abnormalities in the nervous system.

In fact, there are numerous studies in neuroscience and clinical studies that demonstrate the relationship between some neurological and psychiatric diseases with impaired eye and/or cephalic movements. This is disclosed, for example in "Noninvasive Measurement of Head Posture "R. Cerny et al. (2006). The 11th Danube Symposium - International otorhinolaryngological Congress, Bled, pages 39-42.

The abnormality of said eye and/or cephalic movements indicates an alteration of nervous system, and they are present in diseases such as dementias (Alzheimer's, frontotemporal dementia ...), Parkinson's disease, parkinsonism, schizophrenia, obsessive compulsive disorder, anxiety, depression, bipolar disorder, multiple sclerosis, ataxia, ADHD (attention deficit hyperactivity disorder), autism, Tourette's syndrome, dyslexia and dizziness. The following scientific articles describe these abnormal movements:
- *"*Eye movements in patients with neurodegenerative disorders". T. J. Anderson and M. R. MacAskill. (2013) Nature Reviews, Neurology. Pp. 1 -12;
- *"*Diagnosing Stroke in Acute Vertigo: The HINTS Family of Eye Movement Tests and the Future of the Eye ECG". D. E. Newman-Toker, I.S. Curthoys and G. M.I Halmagyi. (2015) Seminar of Neurology Vol. 35. Num 5, pp. 506-21. doi: 10.1055/s-0035-1564298; and
- *"*Ocular motor disorders". (2014) A. W. and C.J. Lueck. Current Opinion in Neurology. Vol 27, pp. 75-82. Doi:10.1097/WCO.0000000000000054.

Therefore, ocular or/and cephalic movements study is a diagnostic tool.

The most relevant movements for diagnosis include, besides the study of the presence of nystagmus (involuntary eye movements that can be vertical, horizontal and rotational) evoked or spontaneous saccades, slow pursuit, the so called Vestibulo-Ocular Reflex (VORs) as well as the study of the presence of nystagmus evoked or spontaneous. These movements will try to keep the target in the position of greatest visual acuity of the retina, called *fovea.* Table 1 summarizes, below, the different types of movements, its definition and their most relevant measuring parameters.

**Table 1: different ocular movements, definition and measurement parameters used. Latency: time taken from the beginning of the stimulus movement and the beginning of the eye movement. Accuracy: distance between the position of the object and the position gaze. Gain: ratio of the peak velocity of the stimulus and the response velocity. Phase: measurement of temporal synchrony between head movement and eye movement. Preponderance: predominant direction of movement. Amplitude: distance travelled by the eyeball. Corrective saccade: saccadic movement made by error in such movement.**

| **Oculomotor function** | **Definition** | **Most relevant parameters** |
|---|---|---|
| **Study of provoked nystagmus for suppression of the visual fixation** | Several vestibular diseases provoke a horizontal or rotary nystagmus when visual fixation is removed, | Presence or absence |
| | | Direction |
| **Rotational vestibuloocular reflex** | Reflex which moves the eyes to keep the image on the retina during rotational movements of the head. | Gain |
| | | Phase |
| | | Preponderance |
| **Translational vestibuloocular reflex** | Reflex which moves the eyes keeping the image on the retina during lineal movements of the head. | Gain |
| | | Phase |
| | | Preponderance |
| **Optokinetics reflex** | Eye movements produced by an object moving out of visual field. | Gain |
| | | Phase |
| **Visually guided horizontal saccades** | Quick eye movement for modifying the sight line between successive points in a horizontal line for keeping the object focussed in the fovea. | -Duration |
| | | -Maximum speed |
| | | -Amplitude |
| **Visually guided vertical saccades** | Quick eye movement for modifying the sight line between successive points in a vertical line for keeping the object focussed in the fovea. | -Gain |
| | | -Precision |
| | | -Latencia and viability |
| | | -Percentage of corrective saccades corrective removed |
| **Visually guided oblique saccades** | Quick eye movement for modifying the sight line between successive points in a vertical line for keeping the object focussed in the fovea. | - Percentage of premature saccades |
| **Memory guided horizontal saccades** | Saccade generated in a location where the object was previously presented horizontally for keeping the object focussed in the fovea. | -Precision |
| | | -Percentage of error |
| **Memory guided vertical saccades** | Saccade generated in a location where the object was previously presented vertically for keeping the object focussed in the fovea. | -Latency and variability |
| **Horizontal anti-saccades** | Saccades generated in the opposite direction to the target in the horizontal sight line | -Precision |
| **Vertical anti-saccades** | Saccades generated in the opposite direction to the target in the vertical sight line | -Percentage of error |
| | | -Latency and variability |
| **Horizontal smooth pursuit without head movement.** | Eye movement to keep a visual stimulus focused in the fovea while it is moving slowly (less than 45 ° / sec speed) in the horizontal axis. | -Quadratic error |
| | | -Gain |
| | | -Velocity gain |
| | | -Presence of intrusive or corrective saccades. |
| **Vertical smooth pursuit without head movement.** | Eye movement without head movement to keep a visual stimulus focused in the fovea while it is moving slowly in the vertical axis | Differences between the initiation and maintenance phase |
| **Horizontal smooth pursuit with free head movement.** | Eye movement with head movement to keep a visual stimulus focused in the fovea while it is moving slowly (less than 45 ° / sec speed) in the horizontal axis | -Previous parameter |
| **Vertical smooth pursuit with free head movement.** | Eye movement with head movement to keep a visual stimulus focused in the fovea while it is moving slowly (less than 45 ° / sec speed) in the vertical axis | - Degree of head movement in tracking |
| **Task to study the perception of scenes and faces** | Measurement of eye movement to keep focused the object in the fovea while a visual recognition of an image or a face is performed. | Location and fixation sequence |
| | | -Duration of fixation |
| | | -Distance between fixations |

In order to reliably determine if the eye movements of a subject are altered or not, it is appropriate to assess the greatest number of different movements. At the same time, many of these eye movements are subtle, high speed and difficult to quantify, especially saccadic movement then, it is necessary to use precision instruments and automatically process of data analysis, frequently with the help of programmable logic devices, specially computers.

In view of the above, in the state of the art many devices can be found for measuring eye movements and to assist in the diagnosis of these diseases through analysis of data obtained.

Thus, for example, in US 2013 0027665 patent application, a device for eye tracking movement is described. The device comprises an image acquisition module disposed to take a single image of both eyes and an optical means for data transmission. In the devices disclosed in this patent application, the image of both eyes is formed by combining several images of each eye, taken through several different optical paths. In addition, the camera must remain fixed and be located about 20 degrees of visual field system of the patient in order to avoid distortion.

However, it would be desirable to provide a device capable, not only of following the eye movements of both eyes, but also of following only one of them and that could be able, preferably, to follow movements of the right and left eye independently. This would increase its versatility and allow to use eye-tracking devices for people with visual impairment in any of two eyes, for example, glaucoma and/or retrobulbar neuritis, or have completely lost vision in one eye.

It is also important to measure eye movement together with head movement because measurements of the head movement are essential for the study of vestibular disorders, which originate vertigo and differentiate them from diseases caused by alterations in brain parenchyma such as cerebellar lesions, brain stem or some cerebral cortices. On the other hand, head movement measurement is very useful to evaluate if the subject needs to compensate eye movements with head movements just it happens in progressive supranuclear palsy. Measuring head movements also allows to study how a subject performs a visual inspection of an image, something that has been useful in ADHD.

Therefore in the state of art, devices for measuring both eye and head movements can also be found.

For instance, US Patent Application No. US5942.954 discloses an apparatus for analysing the visual and vestibular response (ie, spatial control that determines the sense of balance) of a subject. Said device comprises means for inducing a rotation in the head of a subject, a transducer for measuring the angular moment applied to the subject's head, cephalic-tracking means for measuring the cephalic rotational movement of the subject, an eye-tracking system for at least one eye and a controller for estimating visual and vestibular response of the subject based on movements (cephalic and eye) detected.

Moreover, cephalic-tracking means shown in US Patent No. 5,942,954 US are arranged next to the patient's chin (as shown in Figure 3 of that document). Because of this arrangement, it is necessary to implement a coordinate transformation algorithm to calibrate the device in order to measure cephalic displacement of the subject correctly. Said cephalic-tracking means measure the uncorrelated cephalic movements, caused by the vibration system disposed in the helmet, with the eye movement in order to analyse his/her dynamic vestibular response.

In addition, devices shown in US Patent Application No. US 5942.954 are bulky, cumbersome to use and expensive to manufacture, as they require a motor (or other means) to induce a rotation in the head of a subject.

In the state of the art, other possible locations for cephalic monitoring means are shown, but in all of them it is also necessary to implement transformation algorithms. Some examples of other prior art embodiments are shown in the following documents:
In patent application US 2014 / 0358009A1, a glasses for recording eye movements in the diagnosis of vertigo is described. It has a sensor for head movement and the system records the movement of the eyeballs while the head is settled in a predefined position. However, this system does not allow free eye tracking, ie to measure head movements other than those established by the protocol.

In patent application US 2015/0005587 A1 a glasses for the dizziness diagnosis in emergency situations is disclosed. The glasses have a position sensor and an optical sensor that records eye movement while the head of the patient is maneuverer by the medical team.

In patent application JP2013031631A a device easy to use and placement is placed on a Frenzel goggles in order to analysis eye movement for the assessment of the presence of absence nystagmus palsy when the patient cannot fix the gaze.

Therefore, in the sector there is a need for a device that simultaneously solves the following technical problems:
- To be able to measure the maximum number of eye movements of a subject under study, i.e.: movement "Vestibulo-Ocular Reflex" (VORs), fixing movements, slow tracking movements, nystagmus, reflective saccades, exploration saccades, horizontal, vertical and oblique saccades and antisaccades;
- It is capable of simultaneously measuring eye and head movements;
- To be able to measure, the eye movements of both eyes of a patient, as well as the movements of only one of his/her eyes independently; and
- to be provided with means for tracking cephalic movements which are easy to calibrate. Furthermore, it is also desirable to develop new more compact, portable and easy to use devices than those described in the state of the art.

### BRIEF DESCRIPTION OF THE INVENTION

Therefore, one object of the present invention is to provide a device that addresses the problems of the prior art listed above.

More particularly, the device for synchronized measurement of eye and head movements of a subject under study, according to the present invention comprises:
- a helmet;
- a U-shaped projecting arm, having two side portions and a central portion, linked to the helmet by passive joints with at least two degrees of freedom, rotational and translational,
- at least one camera and a lighting system, provided in the central portion of the projecting arm;
- a motion sensor provided with fixation means;
- an emitter of visual stimuli sequences; and
- a processing means which receives the images taken by the camera and the data taken by the motion sensor and quantifies from said images and said data, the cephalic and eye movements made by the subject;
the device being characterized in that:
- it comprises a hub provided with a single input connection that receives the images taken by the camera or cameras and the data taken by the motion sensor and electrically powers the camera or cameras, the lighting system and the motion sensor; said hub being also provided with a single output connection that transmits the images taken by the camera or cameras and the data taken by the motion sensor to the processing means; and in that
- The motion sensor is arranged in a glabellar region of the subject.

The operation of the device according to the invention is as follows: the medical staff who studies the cephalic and eye movements manually set the motion sensor, firstly, to the glabellar region of a subject under study.

This location is particularly suitable for the purposes of the present invention because the glabellar region is a natural symmetry axis of the two eyes of a subject. By placing on it the motion sensor is able to measure the rotational movement of the head precisely because measurement would not be affected by possible slides of the helmet with respect to the head, nor by the relative sliding of the sensor itself when contacting with hair the subject.

Thus, calibration is facilitated by using a pattern of at least nine correlative visual stimuli displayed to the subject for at least two seconds. This allows the processing means to quantify subsequently easily and more reliably, what have been the head movements made by the subject without using transformation algorithms related to eye movements and correct gaze's deviations taking into account the cephalic rotation.

Later, the helmet is firmly attached to the subject's head and the passive joints of the arm are adjusted using passive joint in order to settle the camera in a position that does not obstruct the visual field of the subject under study. The spatial position of the camera is controlled manually by medical staff.

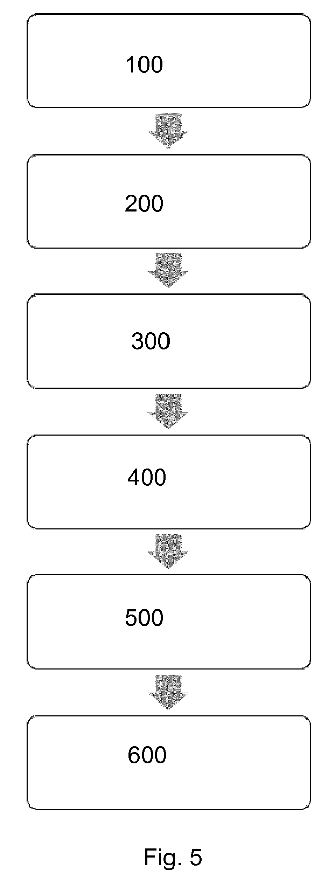

Then the camera, the motion sensor and the lighting system that illuminates at least one of the two subject's eyes are turned on so that the camera can capture clear images of the eye (or eyes) of the subject. The emitter then emits predefined sequences of visual stimuli which are watched by the subject. The images taken by the camera and the data taken by the motion sensor while the subject watches the visual stimuli sequences are synchronized in the hub and transmitted to the processing means. Finally, the processing means analyses the images taken by the camera and the data taken by the motion sensor, and based on them, quantifies the cephalic and eye movements made by the subject (Fig. 5).

Also, the fact that the device according to the invention is provided with a hub allows sending all the information collected (ie, both the images taken by the camera, and the data taken by the motion sensor) to processing means through a single connection.

Moreover, this avoids the need of providing the device with other specific connections, intended to electrically power the camera or cameras, the lighting system and motion sensor. This improves the portability of the device, makes it more compact, and simple to use because it is not required to make multiple power and data transmission connections each time you want to use the device.

In a preferred embodiment of the invention, the visual stimuli sequences emitter is a computer screen, laptop, a television or a tablet device. The visual stimuli sequences emitter is preferably provided with a software and / or programmable hardware elements that allow to configure visual stimuli sequences (ie, defining among other parameters, the visual stimuli present in each sequence, the duration of the stimuli sequence, the duration of the appearance of each visual stimulus and type of movement and direction of movement of each stimulus in the sequence).

Also, the processing media are preferably provided with software and / or hardware elements. More preferably, the hardware elements are programmable logic devices such as desktop computers, laptops, tablet type devices or smart phones.

The lighting system is preferably adjustable in intensity.

In a preferred embodiment of the invention, the camera is movable along a direction parallel to the central portion of the projecting arm. In another preferred embodiment, the camera is disposed on a carriage which is slidably mounted on at least, one rail provided on the central portion of the projecting arm.

With this arrangement, the camera can move in the direction of the rail (or rails), which allows to make both binoculars measurements, as well as single eye measurements of the right eye and single eye measurements of the left eye.

The side portions of the projecting arm are preferably provided with supports or grooves, described later in this specification, to place in the visual field of the subject, but out of sight of the camera or cameras, an element that prevents gaze fixing, such as Frenzel's glasses, so it is possible to perform vestibular tests.

### BRIEF DESCRIPTION OF THE DRAWINGS

Figure 1 is a schematic view of a possible embodiment of the device according to the invention;
Figure 2 is a schematic view showing further details such as the location of the motion sensor of the embodiment shown in Figure 1;
Figure 3 is a schematic view showing an alternative embodiment of the articulated arm of a device according to the invention;
Figure 4 is a schematic view of another alternative embodiment of the invention, where the support which holds the element that prevents gaze fixation, such as a Frenzel crystal, is shown;
Figure 5 is a flowchart which describe the methodology to use the device of the invention.

### DETAILED DESCRIPTION OF THE INVENTION

Below, a specific embodiment of the invention, given as illustrative and non-limiting example, with reference to the accompanying figures is described.

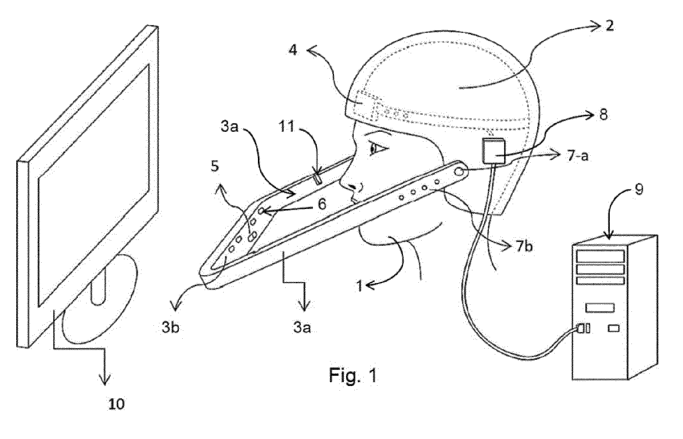

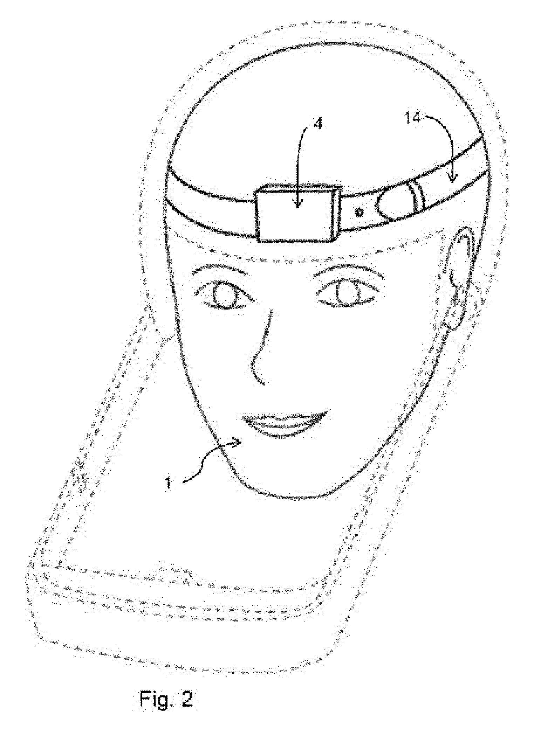

In Figures 1 and 2 a possible embodiment of the device according to the invention is shown. In this embodiment, the movement sensor 4 is fixed to the glabellar region of the subject 1 under study by fastening straps 14.

Subsequently, the helmet 2 is held on the head of the subject 1, being the movement sensor 4 inside the helmet 2. In this embodiment, the helmet is provided with a projecting arm formed by two lateral portions 3a and a central portion 3b. A first passive joint 7a, in the form of an adjustable screw, allows to adjust the relative inclination between the helmet 2 and the lateral portions 3a of the arm (first degree of freedom), whereas a second passive joint 7b, in the form an adjustable screw, which can be inserted through different holes provided in the lateral portions 3a allows adjusting the length of lateral portions 3a (second degree of freedom) of the projecting arm.

The central portion 3b of the arm is provided with at least one camera 5 and a lighting system 6, formed in a preferred embodiment, by intensity adjustable LEDs. For best performance of the device of the invention, the projecting arm is placed by first passive joint 7a, in a position in which the camera 5 focus directly to the eyes of the subject 1.

Also, in a preferred embodiment of the invention the camera 5 is an infrared camera, such as Full HD Model ELP-USBFHD01M Lt ELP-L36 camera or a e-CAM40_CUMI4682_MOD - 4MP camera by e-con Systems Lt.

In this embodiment of the invention, the visual stimuli emitter 10 is a monitor of a desktop computer. Said monitor 10 emits visual stimuli sequences after the movement sensor 4 and helmet 2 are properly placed on the head of the subject 1.

In this embodiment, visual stimuli are different geometric shapes that move, for a predetermined period of time, along a given direction of the monitor 10. The sequences of visual stimuli are configured using software that allows to set, among other parameters, the visual stimuli of each sequence, duration of stimulus sequence, duration of the appearance of each visual stimulus and type of movement and direction of movement of each stimulus in the sequence.

The movement sensor 4, the camera 5 and the lighting system 6 are activated when the subject 1 starts to observe the sequences of visual stimuli displayed on the monitor 10. Also, motion sensor 4, the camera 5 and the lighting system 6 are connected through a first standard USB 2.0 connector, or more preferably, through a standard USB 3.0 connector, to the signal hub 8. Moreover, said signal hub 8 transmits (by a second standard USB 3.0 or 2.0 connector) the images taken by the camera 5 and the data collected by the movement sensor 4 to the processing device (in this embodiment, a desktop computer 9) while the subject is watching the visual stimuli sequences.

The lateral portions 3a of the projecting arm are provided with grooves 11 for accommodating a Freznel crystal or other element which prevents the gaze fixation of the subject, allowing to use the device of the invention to perform vestibular tests, that are described below.

In this embodiment of the invention, the subject 1 watches visual stimuli, in the form of geometric figures, which appear randomly in the monitor 10 and move along different directions.

For measuring the horizontal saccades, a geometric figure will appear randomly on the left or the right along a horizontal axis is shown in the monitor 10.

For measuring the vertical saccades, a geometric figure will appear randomly along a vertical axis is shown in the monitor 10.

For measuring the oblique saccades, a geometric figure will appear randomly along an oblique axis in the monitor 10.

To measure saccades guided by memory, the subject 1 must move the gaze to a predefined point of the monitor 10 which the subject 1 must remember without any visual stimulus in monitor 10.

To measure vertical / horizontal anti-saccades, a geometric figure is shown in the monitor 10 and it will appear randomly along a vertical / horizontal axis. The subject 1 has to look (i.e., move their eyes) at the opposite direction in which the visual stimulus appears.

For measuring vertical / horizontal smooth pursuit eye movement, a geometric figure that moves along a vertical / horizontal direction along a straight / sinusoidal trajectory is shown on the monitor 10. This test can be performed with or without head movement of the subject 1.

For carrying out a head impulse I test, a rotacional 30 degrees movement is performed to the head of the subject 1, while this subject 1 fixes the gaze on a point displayed on the monitor 10.

The devices according to the invention are also able to assess the vestibular response of a subject 1 through the following tests:
For studying the spontaneous nystagmus, a Freznel crystal or the like is placed on the support 16 to avoid visual fixation. Later, the absence or presence and the direction of nystagmus, if present, is checked in the subject 1.

For studying the vestibulo-ocular reflex (VOR), a rotational movement of about 30 degrees is performed on both sides and in several occasions to the head of the subject 1, while the subject 1 is asked to keep the eyes at a particular point of the monitor.

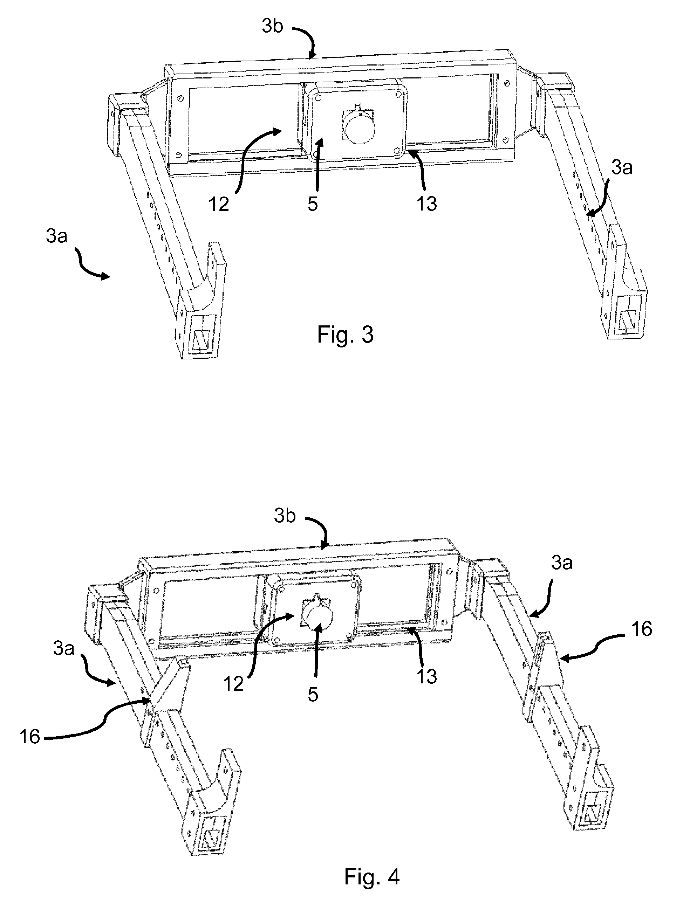

Also, figure 4 shows an alternative embodiment of the invention that allows to perform the Dix-Hallpike (test to trigger the nystagmus with the head movement) manoeuvre. For doing it, an element that avoids gaze fixation such as a Freznel glass is placed on the supports 16. A rotational movement of about 45 degrees.is performed to the head of the subject 1 and he/she is quickly lied down on a couch and the eye movements of the subject 1 are observed for several seconds.

Figure 3 shows another alternative embodiment of the device of the invention in which the camera 5 is arranged on a carriage 12 slidably mounted on two rails 13 provided in the central portion 3a of the arm.

In Figure 5 shows a flow chart describing the method of use of a device according to the invention for synchronized measurement of eye and head movements of a subject. Said method comprises the following steps:
- Fixation (step 100) of the motion sensor 4 through fastening straps 14 for fixing the motion sensor to the glabellar region of the subject 1 under study;
- Holding (step 200) of the helmet 2 to the subject's head 1 under study.
- Adjustment (step 300) of the passive joints 7a and 7b for positioning the camera or cameras 5 in a position that does not interfere the visual field of the subject 1;
- Activation (step 400) of the camera or cameras 5, the motion sensor 4 and the lighting system 6;
- Performance (step 500) of the various tests. Said tests include performing measurements of eye and head movements of the subject 1; and
- Processing (step 600) the data obtained from the camera or cameras 5 and of the motion sensor 4 in the processing means 9.

The method of use of the device according to the invention may also further include the step of introducing, in the visual field of the subject 1 but out of the visual field of the camera or cameras 5, an element that prevents visual fixation, preferably a Freznel crystal.

Although the present invention has been described with respect to preferred embodiments thereof it will be appreciated from the following the description that various combinations of elements, variations or improvements thereof can be made, which are within the scope of the invention, that is defined exclusively by the following set of claims.

### LIST OF NUMERICAL REFERENCES USED IN THE FIGURES

- (1) Subject under study;
- (2): Helmet;
- (3a): Lateral portion of the arm;
- (3b): Central portion of the arm;
- (4): Motion Sensor;
- (5): Camera;
- (6): lighting system;
- (7a): First passive joint;
- (7b): Second passive joint;
- (8): Signal hub;
- (9): processing means;
- (10): Emitter of visual stimuli sequences;
- (11): Groove for Freznel crystal;
- (12): carriage;
- (13): Rail;
- (14): fastening straps for motion sensor;
- (16): Support for place an element that prevents visual fixation;
- (100): Fixation step of the motion sensor;
- (200): Holding step of the helmet;
- (300): Adjustment step of the passive joints;
- (400): Activation step of the camera or cameras, motion sensor and the lighting system;
- (500): Performance step to carry out the different tests; and
- (600) Processing step to process of the data obtained.

### BIBLIOGRAPHY

[1] Noninvasive Measurement of Head Posture, Cerny R. et al. (2006). The 11 th Danube Symposium - International Otorhinolaryngological Congress, Bled, páginas 39-42.
*[2]* Eye movements in patients with neurodegenerative disorders. T. J. Anderson and M. R. MacAskill. (2013) Nature Reviews, Neurology. Pp. 1 -12.
*[3]* Diagnosing Stroke in Acute Vertigo: The HINTS Family of Eye Movement Tests and the Future of the "Eye ECG". D. E. Newman-Toker, I.S. Curthoys and G. M.I Halmagyi. (2015) Seminar of Neurology Vol. 35. Num 5, pp. 506-21. doi: 10.1055/s-0035-1564298.
[4] Ocular motor disorders. (2014) A. W. and C.J. Lueck. Current Opinion in Neurology. Vol 27, pp. 75-82. Doi:10.1097/WCO.0000000000000054.

## Claims

1. Device for synchronized measurement of eye and head movements of a subject (1), under study, said device comprising:
- a helmet (2);
- a U-shaped projecting arm (3a, 3b), which has two lateral portions (3a) and a central portion (3b) attached to the helmet (2) by passive joints (7a, 7b) with, at least, two degrees of freedom, rotational and translational;
- at least one camera (5) and a lighting system (6), provided in the central portion (3a) of the projecting arm (3a, 3b);
- a motion sensor (4) provided with fixation means (14);
- an emitter (10) of visual stimuli sequences; and
- processing means (9) which receive the images taken by the camera (5) and the data taken by the motion sensor (4) and quantifies, from said images and data, the cephalic and eye movements made by the subject (1);
the device being **characterized in that**:
- It comprises a signal hub (8) provided with a single input connection that receives the images taken by the camera or cameras (5) and the data taken by the motion sensor (4) and electrically powers the camera or cameras (5), the lighting system (6) and the motion sensor (4); said signal hub (8) being also provided with a single output connection that transmits the images taken by the camera or cameras (5) and the data taken by the move sensor (4) to the processing means 9); and **in that**
- the motion sensor (4) is arranged in the glabellar region of the subject (1).

2. Device according to claim 1, **characterized in that** the emitter (10) of visual stimuli sequences is a desktop computer monitor, laptop or television screen, or a type tablet device.

3. Device according to any of the preceding claims, **characterized in that** it has at least one camera (5) slidably movable along a direction parallel to the central portion of the projecting arm.

4. Device according to claim 3, **characterized in that** at least one camera (5) is placed on a carriage (12), slidably mounted on at least one rail (13) provided in the central portion (3b) of the arm (3a, 3b).

5. Device according to any of the preceding claims, **characterized in that** the lateral portions (3a) of the arm (3a, 3b) are provided with supports (16) for placing in the visual field of the subject (1), but outside the visual field of the camera or cameras (5), an element to avoid the gaze fixation.

6. Device according to any of the preceding claims, **characterized in that** the lateral portions (3a) of the arm (3a, 3b) are provided with grooves (11) for placing in the visual field of the subject (1) but outside the visual field of the camera or cameras (5) an element to avoid the gaze fixation.

7. Device according to claim 5 or claim 6, **characterized in that** the element that prevents gaze fixation is a Frenzel crystal.

8. Device according to any of the preceding claims, **characterized in that** the lighting system (6) is adjustable in intensity.

9. Device according to any of the preceding claims, **characterized in that** the processing means (9) are provided with elements of software and / or hardware.

10. Device according to claim 9, **characterized in that** the hardware elements are a desktop, a laptop, a tablet type device or a smart phone.

11. Method of use of a device according to any of claims 1 to 10 for the synchronized measurement of eye and head movements of a subject, **characterized in that** it comprises the following steps:
- Fixation (100) of the motion sensor (4) through fastening straps (14) for fixing the motion sensor to the glabellar region of the subject (1) under study;
- Holding (200) of the helmet (2) to the subject's head (1) under study;
- Adjustment (300) of the passive joints (7a) and (7b) for positioning the camera or cameras (5) in a position that does not interfere the visual field of the subject 1;
- Activation (400) of the camera or cameras (5), the motion sensor (4) and the lighting system (6);
- Performance (500) of the different measurements of eye and head movements of the subject (1); and
- Processing (600) the data obtained from the camera or motion sensor (5) and (4) of movement in the processing means (9).

12. Method according to claim 11, **characterized in that** it further comprises the step of introducing in the visual field of the subject (1) but outside the visual field of the camera or cameras (5), an element that prevents visual fixation, preferably a Freznel glass.
